## Europäisches Patentamt
## European Patent Office
## Office européen des brevets

(19)

(11) Numéro de publication: **0 145 563**
**B1**

(12) **FASCICULE DE BREVET EUROPÉEN**

(45) Date de publication du fascicule du brevet:
**10.08.88**

(51) Int. Cl.⁴: **A 61 B 3/12,** A 61 F 9/00

(21) Numéro de dépôt: **84402345.7**

(22) Date de dépôt: **16.11.84**

(54) **Ophtalmoscope catadioptrique à balayage.**

(30) Priorité: **21.11.83 FR 8318512**

(43) Date de publication de la demande:
**19.06.85 Bulletin 85/25**

(45) Mention de la délivrance du brevet:
**10.08.88 Bulletin 88/32**

(84) Etats contractants désignés:
**DE GB**

(56) Documents cité:
**EP-A-0 044 770**
**US-A-3 698 099**
**US-A-4 091 814**
**US-A-4 213 678**

**IEEE TRANSACTIONS ON BIOMEDICAL ENGINEERING, vol. BME-28, no. 7, juillet 1981, pages 488-492, IEEE, New York, US; R.H. WEBB et al.: "Scanning laser ophthalmoscope"**

(73) Titulaire: **CENTRE NATIONAL DE LA RECHERCHE SCIENTIFIQUE (CNRS), 15, Quai Anatole France, F-75007 Paris (FR)**

(72) Inventeur: **Cohen Sabban, Joseph, 31, rue de Paris, F-94100 Orsay (FR)**
Inventeur: **Rodier, Jean- Claude, 6, allée des Bathes, F-91940 Ulis (FR)**
Inventeur: **Roussel, André, 23, avenue Pasteur, F-91800 Brunoy (FR)**
Inventeur: **Simon, Jacques François Henri, 84, rue d'Alleray, F-75015 Paris (FR)**

(74) Mandataire: **Plaçais, Jean- Yves, Cabinet Netter 40, rue Vignon, F-75009 Paris (FR)**

## Description

L'invention concerne les techniques d'examen de l'oeil, et en particulier du fond de l'oeil.

Les dispositifs utilisés à cet effet, généralement appelés ophtalmoscopes, servent à différentes sortes d'examens: ceux-ci vont de la simple observation de la rétine à des applications plus sophistiquées comme l'angiographie en fluorescence, l'examen mono ou polychromatique, et la microophtalmoscopie. En ce qui concerne les examens classiques de la rétine, ce type d'ophtalmoscopie s'effectue avec un champ moyen de 20 à 30°. Enfin, quelle que soit l'application, le niveau des performances atteintes par un ophtalmoscope est défini essentiellement par sa résolution.

Les appareils les plus performants actuellement disponibles sont les rétinographes, qui associent un ophtalmoscope à un appareil photographique. Ceux-ci ne permettent pas un examen visuel direct, et s'accommodent difficilement d'un examen dynamique de la rétine, étant limités techniquement à une fréquence de 3 ou 4 vues par seconde.

Par ailleurs, il est couramment admis en ophtalmologie que tout ophtalmoscope doit obéir à la règle de Gullstrand.

L'application de cette règle consiste à utiliser des parties différentes de la cornée et du cristallin pour éclairer d'une part et observer d'autre part la rétine. Cette règle limite la résolution, et la possibilité de détecter de petits détails.

Des expériences ont été tentées il y a quelques années dans le but d'associer un rétinographe à un tube vidicon, afin de visualiser les structures rétiniennes sur un écran de télévision. Ces expériences n'ont pas fourni les résultats escomptés, notamment parce que la résolution obtenue est faible comparée à celle de la rétinographie photographique.

Plus récemment, le Brevet américain US-A-4 213 678, de POMERANTZEFF et WEBB, a proposé un ophtalmoscope à balayage. Cet appareil constitue une transposition de la technique du point volant ("flying spot") connue en télévision. Cette technique réside en une analyse par balayage électronique au standard de la télévision d'un objet transparent, par exemple un cliché photographique dans le télécinéma. Ce Brevet des ETATS-UNIS enseigne l'usage d'un balayage entièrement optique, effectué par des moyens mécaniques, que permettent de réaliser la déviation à deux dimensions d'un faisceau lumineux tel qu'un faisceau laser à travers une pupille instrumentale réduite comparée à la pupille de l'oeil. L'appareil collecte alors à pleine pupille le signal optique réfléchi en un point quelconque, quelle que soit l'incidence, c'est-à-dire dans le champ de l'instrument qui est typiquement de 30°.

Appliquaant la règle de Gullstrand, cet ophtalmoscope à balayage antérieur souffre d'une part de la limitation imposée à la résolution de par l'usage d'une pupille instrumentale d'illumination réduite. D'autre part, il utilise un collecteur de faisceaux d'observation qui assure la reprise du faisceau sinstantané dans la totalité du champ de l'instrument. Il en résulte une très grande sensibilité au reflet et à la lumière parasites, auxquels le Brevet des ETATS-UNIS précité cherche à remédier par différents moyens optiques, tels que l'utilisation d'une lumière polarisée.

La présente invention vient offrir un ophtalmoscope à balayage possédant une structure différente, qui permet d'accéder à de meilleures performances.

Un premier but de l'invention est fournir un ophtalmoscope à balayage n'appliquant pas la règle de Gullstrand précitée, et couramment admise dans les ophtalmoscopes classiques.

Un autre but de l'invention est de fournir un ophtalmoscope à balayage possédant un faisceau optique de fiable étendue tant en son trajet aller d'illumination qu'en son trajet retour d'observation.

Un autre but de l'invention est de fournir un ophtalmoscope à balayage utilisant au voisinage immédiat de l'oeil des moyens de transmission de lumière du type catoptrique, c'est-à-dire essentiellement constitués de miroirs.

Un autre but de l'invention est d'utiliser dans ces mêmes moyens de transmission um miroir sphérique, dont l'astigmatisme à l'égard de la pupille du patient, vu sous invidence non nulle, est compensé par une adaptation particulière des moyens de balayage.

Un autre but de l'invention est d'utiliser des moyens de transmission de lumière à l'oeil du patient et des moyens de balayage qui sont communs pour le trajet aller d'illumination et pour le trajet retour d'observation, de façon à réduire la lumière parasite.

Un autre but important de l'invention est de permettre la microscopie rétinienne.

Un autre but de l'invention est de compenser les amétropies oculaires que présente éventuellement le patient, afin d'obtenir à grande pupille des images de qualité.

Un autre but encore de l'invention est de stabiliser le faisceau de retour destiné à l'observation, dont l'étendue gémométrique peut alors être rendue très réduite, tout en permettant néanmoins l'observation d'un champ important du fond de l'oeil.

Ces buts sont atteints selon l'invention grâce à un selon la revendication 1.

Cette disposition permet d'utiliser un faisceau optique de faible étendue tant lors de son trajet aller d'illumination que lors du trajet retour d'observation.

Selon une autre caractéristique avantageuse de l'invention, les moyens optiques de transmission à l'oeil sont du type catoptrique, c'est-à-dire essentiellement constitués de miroirs. Pour différent es raisons, les hommes de l'art n'ont utilisé jusqu'à présent que des systèmes optiques dioptriques, y compris dans la partie de l'ophtalmoscope qui est rapprochée de l'oeil. L'usage de moyens catoptriques permet d'une part de supprimer les images parasites rencontrées avec les systèmes dioptriques. D'autre part, il s'est avéré possible de compenser l'astigmatisme rencontré dans les systèmes catoptriques, comme on le verra ci-après.

De préférence, cette compensation s'effectue selon l'invention de la manière suivante: les moyens optiques de transmission à l'oeil comprennent un miroir sphérique, lequel est utilisé sous incidence non nulle. Les moyens de balayage comportent de leur côté deux déviateurs de faisceau commandés, opérant autour d'axes de rotation respectifs qui sont colinéaires aux focales d'astigmatisme que produirait le miroir sphérique sur l'image d'une pupille de patient ponctuelle, compte tenu de l'incidence non nulle.

Dans un mode de réalisation particulier, les deux déviateurs commandés sont des miroirs pivotants.

Il est souhaitable de conserver une symétrie de fonctionnement entre le trajet aller d'illumination et le trajet retour d'observation, en dehors de leur partie commune.

A cet effet, selon une autre caractéristique intéressante de l'invention, l'ophtalmoscope comporte deux organes compensateurs de réfraction semblables, placés l'un sur le trajet d'illumination en amont de l'organe séparateur, l'autre sur le trajet d'observation en aval de l'organe séparateur. Ceci permet une correction en fonction de l'amétropie éventuelle du patient, une telle correction étant souhaitable lorsque l'on trvaille à grande ouverture de la pupille du patient.

Dans un mode de réalisation, chaque organe compensateur comporte un objectif fixe et un chariot porteur d'un objectif mobile et d'un diaphragme faisant office de pupille d'entrée, les chariots de deux organes compensareurs se déplaçant conjointement et solidairement.

Un simple échange de la lentille placée à l'entrée du compensateur prévu pour le trajet aller d'illumination permet de travailler soit en microscopie rétinienne à balayage, à champ petit, soit en observation à champ moyen, le diaphragme des compensateurs de réfraction étant ajusté en conséquence.

Le bloc de source lumineuse peut être réalisé à partir d'un laser ou d'un arc et de moyens optiques à focalisation, pour générer un point-source lumineux. Ce point est appliqué au compensateur de réfraction à travers la lentille interchangeable. Le transport du faisceau de la source jusqu'au point-source peut se faire à travers une fibre optique, au moins pour le travail en champ moyen. On réalise ainsi une séparation physique entre la source proprement dite et la partie de l'ophtalmoscope qui est rapprochée du patient.

Dans le trajet retour d'observation, un objectif de détection est placé immédiatement en amont du petit diaphragme de détection précité, lequel est placé au foyer de cet objectif, et suivi du photodétecteur.

Selon un autre aspect de l'invention, le plus rapide des deux déviateurs, qui réalise le balayage des lignes, est agencé oscillant, produisant ainsi un balayage sinusoïdal. L'ophtalmoscope comporte en outre des moyens électroniques propres à numériser la sortie du photodétecteur, ainsi qu'à remplir en sens inverse deux mémoires numériques à l'aide des signaux détectés pendant l'aller et le retour d'une période de la sinusoïde. De préférence, l'échantillonnage de la sortie du photodétecteur est effectué à une cadence variable en fonction de la vitesse d'excursion du balayage sinusoïdal.

D'autres caractéristiques et avantages de l'invention apparaîtront à l'examen de la description détaillée ci-après, et de dessins annexés, sur lesquels:

- la figure 1 est une vue d'ensemble des différents blocs fonctionnels constituant un mode de réalisation de l'ophtalmoscope selon l'invention;
- la figure 2 est une autre vue de l'ophtalmoscope de la figure 1, un peu plus détaillée;
- la figure 3 est un diagramme temporel illustrant le remplissage des mémoires numériques en fonction du balayage sinusoïdal, ainsi que la lecture de ces mêmes mémoires;
- la figure 4 est le schéma de principe descircuits électroniques de l'ophtalmoscope selon l'invention; et
- les figures 5 et 6 illustrent de manière plus détaillée les blocs 113 et 114 de la figure 4.

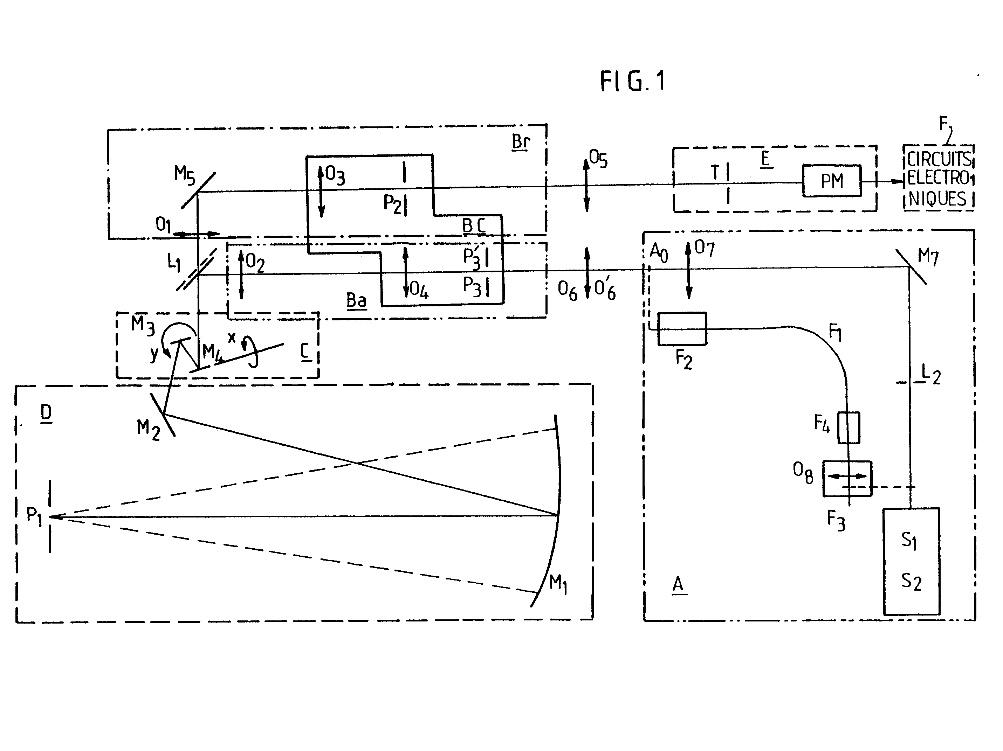

L'ophtalmoscope illustré sur la figure 1 est composé de sous-ensembles, à savoir:

- un bloc de source lumineuse A,
- un compensateur de réfractionà l'aller $B_a$,
- un sous-ensemble de balayage C,
- des moyens de transmission de la lumière à l'oeil, que l'on appellera ci-après ophtalmoscope catoptrique D, compte tenu de leur réalisation à base de miroirs,
- un compensateur de réfraction $B_r$ sur le trajet retour d'observation,
- un bloc de détection et de mesure E, et
- des circuits électroniques F.

Cette structure permet de donner une image de télévision pour une région quelconque de la rétine, ou plus généralement de l'intérieur de l'oeil.

Dans le bloc de source lumineuse A, le faisceau lumineux de base est produit soit par un laser de faible puissance $S_1$, soit par un arc au xénon $S_2$. Ce bloc a pour fonction d'engendrer à partir du faisceau de bas e un point lumineux $A_o$ dont les caractéristiques géométriques et spectrales sont définies par le choix de l'application particulière envisagée: angiographie en fluorescence, examen mono ou polychromatique, microophtalmoscopie, ou ophtalmoscopie à champ moyen de 20 ou 30°.

Dans le cas d'un ophtalmoscope à balayage optique qui fonctionne en examen de champ moyen, il est avantageux que le bloc source A soit réalisé à partir d'une fibre optique $F_1$ possédant par exemple un diamètre de coeur de 6 microns. La fibre optique est maintenue en place par des éléments de fixation $F_2$, $F_3$ et $F_4$. Un objectif $O_8$ focalise le faisceau de base pour lui donner les caractéristiques géométriques désirées.

On peut ainsi dissocier mécaniquement la source (laser ou arc) du reste de l'optique, et par là même alléger l'ensemble tout en réduisant notablement l'encombrement de l'ophtalmoscope proprement dit.

Une variante convient à toutes les applications. Le faisceau de la source $S_1$ ou $S_2$ est appliqué à un miroir de renvoi à 45° $M_7$, qui réfléchit le même faisceau vers un objectif de microscope $O_7$, semblable à l'objectif $O_8$ de l'autre trajet. Dans le cas d'un laser, on interpose avantageusement un diaphragme d'épuration $L_2$ sur le trajet du faisceau laser.

Un faisceau laser associé à un trou de fitrage permet notamment d'offrir la résolution optique nécessaire pour opérer en mode microscopie rétinienne par balayage. Le champ balayé est alors réduit (6 à 8°).

Dans les deux cas, on obtient en sortie du bloc source A une tache élémentaire d'examen $A_o$ que l'on appellera dans la suite point-source.

La suite de la description est placée dans le cas le plus fréquent où l'on examine la rétine elle-même, et non pas un plan de coupe situé à l'intérieur de l'oeil en avant de celle-ci.

En présence d'amétropie du patient (sphère et cylindre), le point-source délivré par le bloc A ne correspond pas nécessairement en position et en surface d'ondes associées - à un conjugué optique de la rétine à examiner. Le compensateur de réfraction $B_a$ permet de compenser ces amétropies.

Avant la compensation proprement dite, le point-source $A_o$ est appliqué à un objectif $O_6$, qui le renvoie à l'infini, produisant ainsi un faisceau de rayons parallèles. Pour la microscopie rétinienne, on utilise un objectif $O_6$ de microscope (grandissement de l'ordre de 20).

Dans le mode de réalisation représenté, le compensateur de réfraction proprement dit commence par un diaphragme $P'_3$ ou $P_3$, qui présente une ouverture de 3 mm ou 1 mm suivant que l'on opère en microscopie rétinienne ou en champ moyen. Il est suivi d'un objectif $O_4$. L'objectif $O_4$ et le diaphragme $P_3$ ou $P'_3$ sont mobiles conjointement en translation axiale le long du faisceau, sur un chariot BC. Plus loin, un autre objectif $O_2$ identique à l'objectif $O_4$ reprend le faisceau. Les diaphragmes $P_3$ ou $P'_3$ sont placés au foyer objet de l'objectif $O_4$. Celui-ci reçoit donc du point-source $A_o$ un faisceau parallèle de section variable en fonction du diaphragme $P_3$ ou $P'_3$ et de l'application envisagée. Par une translation du chariot BC, on amène le point-source dans la position correspondant à une bonne mise au point sur la rétine. La correction de l'astigmatisme du patient est obtenue par adjonction de lentilles cylindriques provenant d'une boîte de verres classiques, et placées au voisinage de $P_3$ ou $P'_3$.

De son côté, l'objectif $O_2$ a pour rôle essentiel de former en son foyer une image réelle de la pupille ($P_3$ ou $P'_3$) au voisinage du couple $M_3$, $M_4$ décrit ci-après. Le couple $O_4$ + $O_2$ assure ainsi un transport de grandissement unitaire de la pupille.

A la sortie du compensateur de réfraction aller $B_a$, le faisceau lumineux arrive sur une lame séparatrice $L_1$, qui le réfléchit à 45° vers les moyens de balayage C.

Il convient de remarquer dès maintenant la symétrie entre le compensateur de réfraction aller $B_a$ et le compensateur de réfraction retour $B_r$. A partir de la lame séparatrice $L_1$, prise en retour et par transmission, le faisceau retour va rencontrer tout d'abord un objectif $O_1$ identique à $O_2$, suivi d'un miroir de renvoi à 45° $M_5$. Après cela, on prend, en sens inverse, un objectif $O_3$ et un diaphragme $P_2$ montés tous deux sur le même chariot BC que l'objectif $O_4$ et le diaphragme $P_3$ ou $P'_3$ auxquels ils sont respectivement identiques. Le diaphragme $P_2$ est muni le cas échéant de lentilles cylindriques correspondant à celles de $P_3$ ou $P'_3$. On va donc retrouver en sortie du compensateur de réfraction retour $B_r$ un faisceau de rayons parallèles, qui a subi exactement le même traitement optique que le trajet aller, depuis l'aval de la lentille $O_6$ ou $O'_6$. C'est la symétrie déjà relevée entre le trajet aller et le trajet retour.

Il est maintenant possible de traiter du trajet optique allant vers le bas à partir de la lame séparatrice $L_1$, sans distinguer suivant qu'il s'agit du trajet aller ou du trajet retour.

Après la réflexion intervenue sur la lame séparatrice $L_1$, le trajet optique rencontre deux miroirs $M_3$ et $M_4$, qui défléchissent le faisceau lumineux autour de deux directions perpendiculaires, de manière à construire une trame constituée de lignes parallèles, semblables à celles produites par le faisceau d'électrons d'un tube de télévision. On peut de la sorte balayer 25 images de 625 lignes chacune par seconde (standard européen).

Le miroirplan $M_4$, solidaire d'un système électromécanique résonnant, assure le balayage lignes, en pivotant autour d'un axe de rotation x. (une variante consisterait à utiliser un miroir polygonal tournant à vitesse constante). Le miroir plan $M_3$, actionné à pivotement en dents de scie assymétriques par un système galvanométrique asservi, reprend la ligne et la distribue en une trame.

Le faisceau ainsi dévié est appliqué à l'ophtalmoscope catoptrique D. Celui-ci commence par un miroirplan $M_2$ permettant un repli des faisceaux pour dégager un espace suffisant répondant aux nécessités pratiques et ergonomiques des observations rétiniennes périphériques. La partie essentielle de cet ophtalmoscope D est un miroir sphérique $M_1$, qui a pour fonction d'assurer la conjugaison pupillaire et la conjugaison du point volant produit par le balayage et de la rétine.

Jusqu'à présent, les dispositifs d'ophtalmoscopie utilisaient à cet effet des optiques dioptriques, source d'images parasites dues aux réflexions sur les dioptres des objectifs qu'elles contiennent. La raison en est sans doute la règle de Gullstrand déjà citée, dont l'application est traditionnelle en ophtalmologie.

La présente intention va directement à l'encontre de cette tradition, en n'appliquant pas la règle de Gullstrand.

De surcroit, l'invention préconise l'usage d'un ophtalmoscope catoptrique, et ce en dépit de l'astigmatisme inhérent à l'usage d'un miroir sphérique fonctionnant sous incidence non nulle. Il a été observé en effet que cet astigmatisme peut être compensé.

Une façon simple de le faire consiste à faire tourner les deux miroirs $M_3$ et $M_4$ autour d'axes de rotation respectifs qui sont, comptetenu de la réflexion en $M_2$, colinéaires aux focales d'astigmatisme que produirait le miroir sphérique $M_1$ sur l'image d'une pupille de patient ponctuelle (c'est-à-dire ramenée à son point central). Ainsi, en produisant un effet équivalent à l'astigmatisme, le système de balayage préconisé permet de réduire considérablement les aberrations aux pupilles. Ceci concerne aussi bien l'aller que le retour du faisceau.

Un autre avantage tiré de cette disposition est qu'après son retour inverse, en sortie des moyens de balayage C, le faisceau lumineux d'observation est immobile. Ceci permet alors une bonne détection de l'information provenant du point rétinien illuminé.

Après avoir traversé en retour la lame séparatrice $L_1$, le faisceau d'observation fixe est alors repris par le compensateur de réfraction $B_r$, identique à $B_a$, avec les mêmes verrez de correction cylindriques.

Comme précédemment indiqué, le chariot mobile BC assure la même correction cylindrique et sphérique dans les deux compensateurs. Il permet des corrections d'amétropie dans une plage de plus ou moins quinze dioptries en sphérique et de plus ou moins cinq dioptries en cylindrique. On obtient alors finalement une image du point volant sur la rétine, image qui est absolument fixe tant en direction qu'en position, quelles que soient les amétropies du patient dans ces plages.

En sortie du compensateur de réfraction retour $B_r$ est placé un objectif $O_5$, qui forme ainsi en son foyer une image réelie immobile et quasi-ponctuelle du point volant dans le champ rétinien balayé.

Le bloc de détection E comporte alors un petit diaphragme T, placé dans le plan de cette image fixe et quasi-ponctuelle de la rétine, afin de réaliser un filtrage spatial de l'information, et ainsi de s'abstraire de la lumière parasite véhiculée par le système et provenant essentiellement des régions antérieures de l'oeil (cornée et cristallin).

Le champ instantané d'observation est donc délimité par ce diaphragme T.

Un photodétecteur PM, de préférence un photomultiplicateur, est alors placé en aval du même diaphragme T. Bien que la position de ce photodétecteur sur l'axe optique ne soit pas critique il est possible de le placer dans le plan de l'image de la pupille donnée par une lentille de champ ajoutée derrière T.

Le champ d'observation est alors réduit au champ instantané du point de balayage. Bien entendu, la taille de la surface sensible du photodétecteur est également choisie pour correspondre à celle de l'image de la pupille du patient. On obtient alors une diminution considérable de la quantité de lumière parasite récoltée, et par là même un gain considérable quant au rapport signal/bruit, ce qui traduit la qualité de l'ensemble de l'ophtalmoscope.

Les signaux de sortie du photomutiplicateur PM sont ensuite appliqués à des circuits électroniques F que l'on décrira plus loin. Ceux-ci sont naturellement reliés aux miroirs oscillants $M_3$ et $M_4$, pour assurer la entre le balayage optique et le balayage d'un moniteur de télévision chargé de la visualisation de l'image de la rétine.

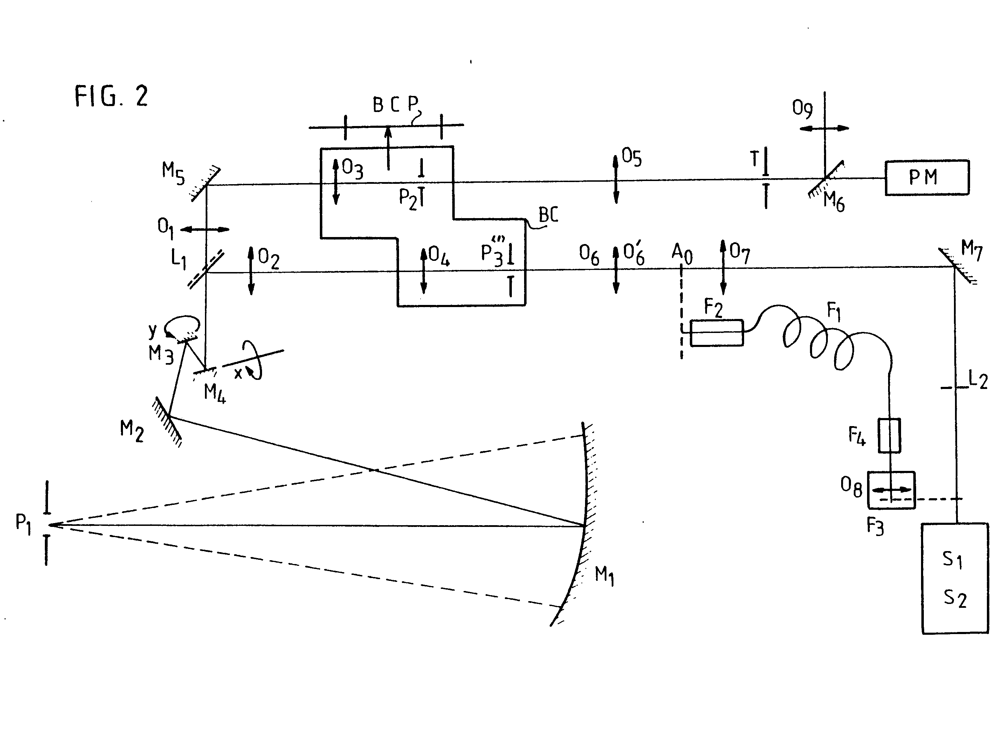

Il convient de commenter brièvement la figure 2. Celle-ci diffère assez peu de la figure 1. On notera en particulier l'illustration du fait que la fibre optique $F_1$ permet une séparation de la source proprement dite et du reste de l'appareil.

L'association du chariot BC des deux compensateurs de réfraction à un organe indicateur tel qu'un curseur potentiométrique BCP permet pour l'opérateur une visualisation en dioptrie de la correction sphérique couramment appliquée. On se rappellera que la correction cylindrique est définie par des verres prélevés dans une boîte, comme le font les ophtalmologistes, et placés au niveau des deux diaphragmes $P_2$ et $P_3$ (ou $P'_3$).

Une dernière différence entre les figures 1 et 2 est à noter entre le petit diaphragme de sortie T et le photomultiplicateur PM est placé un miroir à réflexion partielle et/ou sélective en longueurs d'onde $M_6$, qui permet la reprise du faisceau vers un objectif $O_9$, par exemple pour l'observation ou la visualisation directe de l'image d'un point de la rétine ou encore pour une mesure de l'intensité du faisceau reçu. De manière connue, l'intensité du faisceau émis peut être obtenue d'après la quantité de lumière traversant directement la lame séparatrice $L_1$.

La suite de la description détaillée se place dans le cas où le balayage produit par le miroir $M_4$ est sinusoïdal.

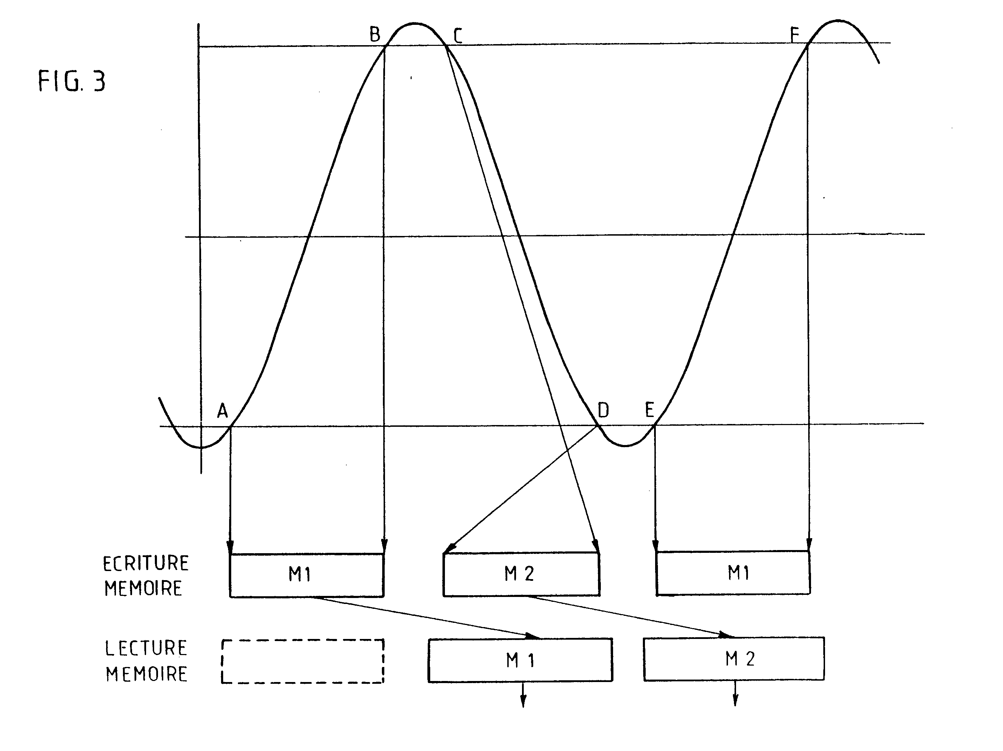

Un tel balayage optique sinusoïdal est illustré sur la figure 3.

Pendant la partie AB d'une demi-période montante de la sinusoïde, on va remplir une mémoire $M_1$ à partir d'échantillons du signal détecté, obtenu en sortie du photomultiplicateur PM. Pendant la demi-période descendante CD qui suit immédiatement, on va remplir une mémoire $M_2$. Cependant, le remplissage de la mémoire $M_2$ se fait dans l'ordre inverse des adresses par rapport à celui de la mémoire $M_1$. Ensuite, avec la première demi-période EF qui suit, on remplit à nouveau la mémoire $M_1$, et ainsi de suite.

Pour la lecture des mémoires, on procède comme suit pendant le remplissage de la mémoire $M_2$, les informations precédemment introduites dans la mémoire $M_1$ sont lues, dans l'ordre normal des adresses. Pendant le second remplissage de la mémoire $M_1$ (créneau EF), c'est alors le contenu de la mémoire $M_2$ qui est lu, toujours dans l'ordre normal des adresses, donc en sens inverse de l'ordre de remplissage de la mémoire $M_2$. On réalise ainsi un balayage par ligne qui sont toutes explorées dans le même sens, au standard de la télévision. Une variante simple consisterait à remplir les deux mémoires $M_1$ et $M_2$ dans le même sens de l'adressage, et à procéder à la lecture de ces mêmes mémoires en des sens inverses d'adressages l'une par rapport à l'autre.

La demi-période du balayage lignes est donc synchrone d'une ligne vidéo. Le balayage horizontal sinusoïdal s'effectue alors à 8 kHz. A partir de là, on va obtenir un signal vidéo standard de 625 lignes et de 50 trames par seconde, avec un balayage vertical en dents de scie, à cinquante Hz. Le balayage peut être entrelacé, en utilisant un décalage d'une demi-ligne entre chaque trame et la suivante.

Enfin, l'échantillonnage du signal de sortie du photodétecteur PM s'effectue à une cadence instantanée proportionnelle à la vitesse du balayage horizontal, ce qui permet de supprimer la distorsion liée au caractère sinusoïdal de ce balayage.

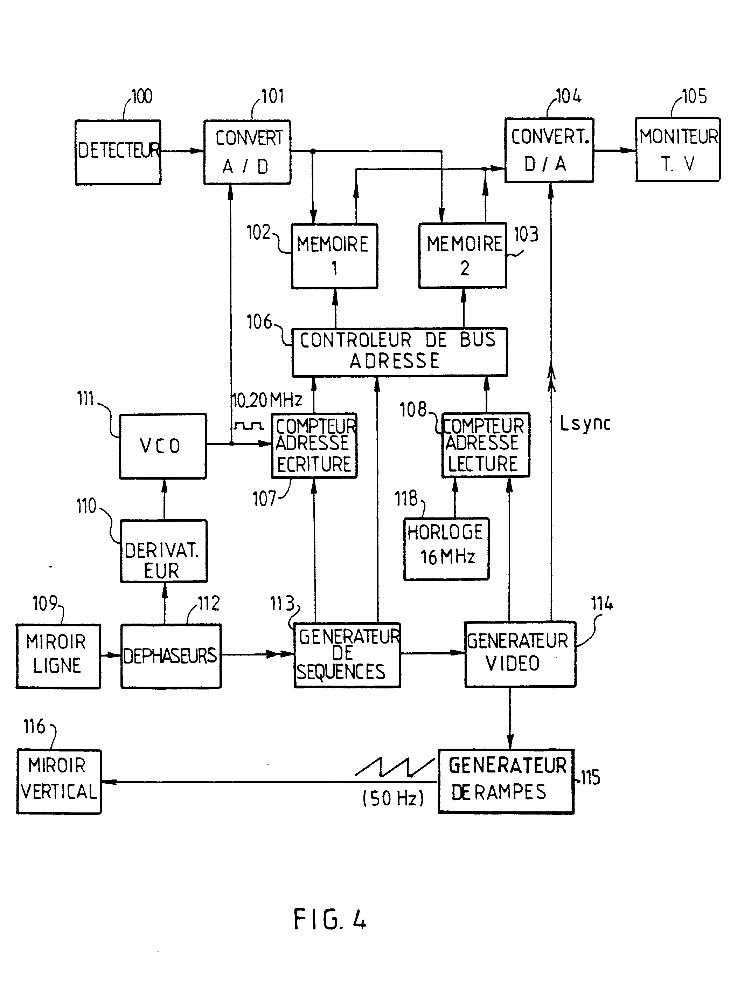

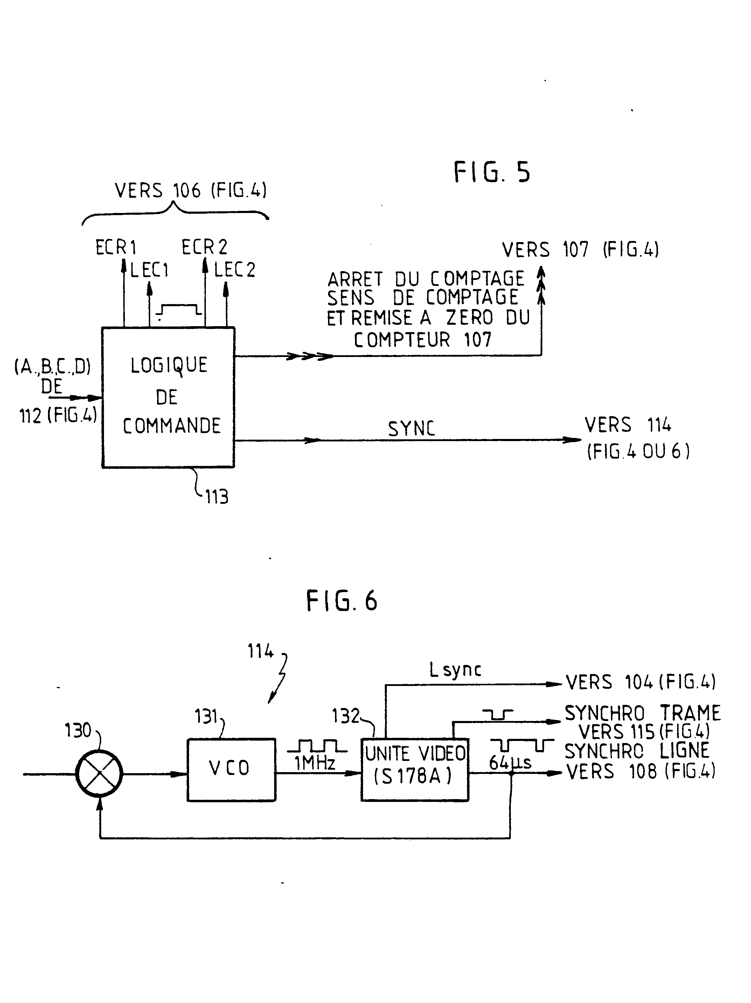

Il est maintenant fait référence à la figure 4. Le photodétecteur PM est maintenant désigné par la référence 100. Son signal électrique de sortie est appliqué à un convertisseur analogique numérique 101, qui présente également une entrée de commande d'échantillonnage.

Comme précédemment indiqué, le miroir de lignes $M_4$, noté maintenant 109, oscille de manière résonnante à 8 kHz. Il fournit donc la référence de position et de synchronisation. Il est muni d'un transducteur donnant une indication de sa position sous forme électrique. Compte tenu du caractère résonnant de la commande du miroir de lignes, un écart peut exister entre ce signal de position et la position réelle du miroir. Le signal de sortie du miroir lignes est ainsi appliqué à un ensemble de déphaseurs 112, qui ont tout d'abord pour fonction de compenser cet écart entre la position réelle du miroir et celle fournie par son signal de position. Les déphaseurs en question sont également agencés pour déterminer les points A B C et D tels qu'illustrés sur la figure 3. L'homme de l'art comprendra que ceci peut être réalisé par des déphaseurs (retards différents appliqués au signal sinusoïdal), suivis d'autant de comparateurs.

Un signal simplement corrigé par le premier déphaseur du bloc 112 pour indiquer la position réelle du miroir est appliqué à un circuit 110 formant dérivateur. S'agissant d'un signal sinusoïdal, sa dérivée est également sinusoïdale, et décalée de $\frac{\pi}{2}$. Le dérivateur 110 peut donc comprendre un simple déphaseur. Il fournit en sortie une tension proportionnelle à la vitesse instantanée de rotation du miroir lignes, tension qui commande un oscillateur 111 lequel est précisément du type contrôlé par une tension. L'oscillateur 111 peut alors délivrer des signaux d'horloge dont la cadence est comprise entre 10 et 20 MHz, tout en demeurant proportionnelle à la vitesse de rotation du miroir lignes. Ce signal d'horloge est appliqué à un compteur-décompteur d'adresses d'écriture 107 et au convertisseur analogique numérique 101, sur son entrée de commande d'échantillonnage.

Il est également prévu un compteur-décompteur d'adresses de lecture 108, mais utilisé en comptage seulement.

Il est maintenant fait référence à la figure 5.

Des informations représentant la position temporelle des points tels que A, B, C, D sur la figure 3 sont appliquées à un générateur de séquence 113 dont un exemple de réalisation est illustré sur cette figure 5. Celui-ci comprend essentiellement une logique de commande, qui établit à partir de A, B, C, D des signaux $ECR_1$, $LEC_1$, $ECR_2$ et $LEC_2$ représentant respectivement l'écriture dans la mémoire 1, la lecture dans la mémoire 1, l'écriture dans la mémoire 2 et la lecture dans la mémoire 2.

La logique de commande fournit aussi d'autres signaux destinés à l'arrêt du comptage et à la remise à 0 du compteur décompteur d'adresses d'écriture 107 ainsi qu'à la détermination de son sens de comptage pour obtenir le fonctionnement décrit à propos de la figure 3. Elle fournit aussi un signal SYNC pour synchroniser le générateur vidéo 114, à partir des points milieux des segments tels que BC et DE (figure 3).

Un schéma détaillé du générateur vidéo 114 est donné sur la figure 6. Celui-ci comporte un asservissement de phase permettant de synchroniser la ligne vidéo avec le balayage horizontal. Il est constitué d'un comparateur de phases dont les entrées reçoivent les signaux issus du bloc 113 et d'une unité vidéo 132. La sortie du comparateur de phases est appliquée à un autre oscillateur commandé en tension 131, opérant à une fréquence nominale de 1 MHz, et délivrant des signaux rectangulaires. Ceux-ci sont appliqués à l'unité de commande vidéo 132, qui peut être un circuit intégré du commerce prévu pour fournir les différents signaux de commande vidéo classiques, à savoir des signaux de synchronisation, ligne, trame et mélangé (trame + ligne) selon les normes CCIR. L'unité vidéo 132 fournit également de purs signaux de synchronisation de trames, qui sont adressés à un générateur de rampe 115, et de purs signaux de synchronisation de lignes, qui sont transmis au compteur-décompteur d'adresses de lecture 108.

Le générateur de rampe 115, qui peut être un intégrateur remis à zéro par les signaux de synchronisation de trames, pilote le balayage en dents de scie asymétrique du miroir vertical noté 116 sur la figure 4.

Le compteur d'adresses de lecture 108 opère normalement à une fréquence d'horloge fixe de 16 MHz définie par une horloge 118. Il est remis à zéro par chaque impulsion de synchronisation de lignes fournie par le générateur vidéo 114.

A l'aide des quatre signaux fonctionnels $ECR_1$, $LEC_1$, $ECR_2$ et $LEC_2$ fournis par le générateur de séquence 113, et des signaux d'adresses fournis en sortie des compteurs 107 et 108 sous forme parallèle, un contrôleur de bus adresses 106 va adresser convenablement la première et la seconde mémoire, notées respectivement 102 et 103.

Plus précisément, le compteur-décompteur d'adresses d'écriture va faire varier dans le sens croissant, et à cadence variable, l'adresse appliquée à la mémoire 1 pour l'écriture, tandis que le compteur 108 va faire varier à cadence fixe l'adresse appliquée à la mémoire 2, dans le sens croissant.

En vue de la phase suivante, il se produira d'une part une interversion des connexions d'adresses entre les deux compteurs et les deux mémoires. D'autre part, intervient un blocage du compteur 107 à la dernière valeur du cycle précédent puis une inversion de sens dudit compteur 107. Ensuite, le compteur 107 fera varier maintenant dans un sens décroissant et à cadence variable l'adresse appliquée à la mémoire 2 pour l'écriture, tandis que le compteur 108 fera varier à cadence fixe l'adresse appliquée à la mémoire 1 dans le sens croissant comme précédemment.

On obtient ainsi, pour l'écriture et la lecture en mémoire, le fonctionnement général déjà décrit à propos de la figure 3.

Les signaux délivrés alternativement par l'une ou l'autre des mémoires 102 et 103 sont appliqués à un convertisseur numérique analogique vidéo 104, qui reçoit aussi comme on l'a déjà vu les signaux $L_{sync}$ provenant du générateur vidéo 114.

La sortie du convertisseur 104 présente alors tous les signaux nécessaires pour la commande d'un moniteur de télévision classique 105.

On donne ci-après un exemple de réalisation détaillée de la présente invention.

- exemple particulier

### Bloc de source lumineuse A

$S_1$ = laser Argon Spectra Physics à 488 nm (bleu) ou 514,5 nm (vert); ou laser He - Ne Hughes à 632,8 nm;
ou $S_2$ = arc Xenon (Cermax Xe illuminates)
$O_7$, $O_8$ = objectif de microscope LEITZ F1 x50, 0,85
$L_2$ = diaphragme d'épuration (laser)
$M_7$ = miroirplan métallisé à 45°

### Compensateur de réfraction aller Ba

$O_4$ = objectif Clairaut (Cerco) f = 50 mm; $\varnothing$u 15 mm
$P'_3$ = 3 mm en microscopie rétinienne
$P_3$ = 1 mm en champ moyen de 20 à 30°
$O_6$ = objectif de microscope Nachet x6, 215 mm
$O'_6$ = objectif de microscope Nachet x19, 215 mm
$O_2$ = objectif Clairaut Cerco f = 50 mm $\varnothing$u = 15 mm

### Balayage optique C

$L_1$ = lame séparatrice n° O3BPLOO5 de Melles Griot à réflexion vitreuse
$M_4$ = miroirplan de General Scanning actionné par système électro-mécanique résonnant à oscillations sinusoïdales (S108)
$M_3$ = miroirplan de General Scanning actionné en dents de scie par système galvanométrique (S116)

### Optique de transmission à l'oeil D

$M_2$ = miroirplan métallisé de renvoi
$M_1$ = miroir sphérique métallisé (aluminium protégé) de rayon 290 mm
$P_1$ = pupille du patient
Retour inverse: compensateur de réfraction $B_R$

$O_1$ = $O_2$
$M_5$ = miroirplan métallisé de renvoi à 45°
$O_3$ = $O_4$
$P_2$ = $P_3$ ou $P'_3$

### Détection E

$O_5$ = objectif Clairaut Cerco f 150 mm, $\varnothing$u = 30 mm
T = diaphragme de filtrage spatial aux dimensions de l'image
PM = photomultiplicateur RTC modèle 150 AVP

### Electronique F

Miroir ligne 109: balayage sinusoïdal à 8 kHz
Miroir Vertical 116: asservi en position selon dents de scie à 50 Hz engendrée en 115
Générateur d'impulsions vidéo 114 circuit intégré S 178A de Siemens , avec boucle d'asservissement en phase (S124, 7474), sur sortie de 113
Générateur de séquence 113: portes TTL 7400, 7402, 7404
Convertisseur analogique numérique 101: convertisseur flash 4 bits 30 MHz (TDG 1021 J de TRW Inc)
Mémoires 102 et 103: chacune une ligne de 800 pixels de 4 bits, pouvant fonctionner à 20 MHz, par exemple 4 boîtiers 93 425A de Fairchild.
Convertisseur numérique analogique 104: 4 bits en 7 ns, comme le modèle HDG 0405 de Analog Devices, à sortie compatible vidéo avec synchronisation.

Moniteur 105: tout moniteur TV 625 lignes, avec ou sans magnétoscope associé.

Compteur d'adresses 107 et 108 10 bits tel que 3 circuits intégrés 74 S 169 (compteur-décompteur) pour chacun.

Contrôleur de bus: 6 circuits LS 244
Déphaseurs: 4 fois 1/4 de TDB 84
Générateur de rampe: 1/4 de TDB 84 et AD 7512

L'ophtalmoscope ainsi obtenu a montré des possibilités très intéressantes, notamment pour l'observation de détails rétiniens, en particulier l'observation dynamique de la circulation sanguine.

Dans le cas d'une observation colorée de la rétine, la source $S_1$ ou $S_2$ émet au moins deux radiations monochromatiques distinctes (ou bien dans au moins deux régions du spectre). Le reste de l'instrument (figure 2) est inchangé, 116 étant un séparateur dichroïque, suivi d'au moins une voie de détection montée comme la première (photomultiplicateur, etc). Un moniteur couleur est alors excité, en vraie ou fausse couleur, selon l'ensemble des signaux détectés.

Il est à souligner par ailleurs que l'ophtalmoscope travaille à grande ouverture de la pupille, et forme des images de celle-ci en différents endroits:
- au voisinage du couple $M_4$, $M_3$
- en $P_2$
- le cas échéant, au niveau du photomultiplicateur PM.

L'homme de l'art comprendra que l'acquisition séparée d'informations passant par deux zones différentes de la pupille permet d'accéder à la stéréoscopie.

## Revendications

1. Ophtalmoscope à balayage, comprenant des moyens d'illumination de l'oeil ainsi que des moyens d'observation de l'oeil ainsi illuminé, lesdits moyens d'illumination de l'oeil comportant un bloc de source lumineuse (A), des moyens de balayage (C) propres à dévier le faisceau produit par le bloc de source lumineuse selon une trame de lignes, et des moyens optiques (D) pour la transmission du faisceau lumineux ainsi dévié à l'oeil du patient, à travers sa pupille, lesdits moyens optiques de transmission (D) étant également utilisés, en sens inverse, pour l'observation, ledit ophtalmoscope comportant en outre des moyens ($L_1$) pour séparer les faisceaux d'observation et d'illumination, et lesdits moyens d'observation comportant un moyen transducteur photoélectrique tel qu'un photomultiplicateur (PM) pour détecter le faisceau d'observation ainsi séparé caractérisé en ce que les moyens de balayage (C), sont également utilisés, en sens inverse, pour l'observation, en ce lesdits moyens séparateurs de faisceaux ($L_1$) sont constitués par un organe tel qu'une lame séparatrice, situé sur le trajet du faisceau d'observation en aval des moyens de balayage (C) en ce que les moyens d'observation comprennent en outre un petit diaphragme de détection (T) conjugué d'un pointsource ($A_o$) produit par le bloc de source lumineuse, et d'ouverture légèrement supérieure à la taille de l'image de ce point-source, et en ce que ledit moyen transducteur photoélectrique est placé en aval dudit diaphragme.

2. Ophtalmoscope à balayage selon la revendication 1, caractérisé en ce que les moyens optiques de transmission à l'oeil (D) sont du type catoptrique ($M_1$), ce qui permet de supprimer les images parasites.

3. Ophtalmoscope à balayage selon la revendication 2, caractérisé en ce que les moyens optiques de transmission à l'oeil comprennent un miroir sphérique ($M_1$), lequel est utilisé sous incidence non nulle, et en ce que les moyens de balayage (C) comprennent deux déviateurs de faisceau commandés ($M_3$, $M_4$) opérant autour d'axes de rotation respectifs (x, y) colinéaires aux focales d'astigmatisme que produirait le miroir sphérique ($M_1$) sur l'image d'une pupille de patient ponctuelle, compte tenu de ladite incidence non nulle.

4. Ophtalmoscope à balayage selon la revendication 3, caractérisé en ce que les deux déviateurs commandés sont des miroirs pivotants ($M_3$, $M_4$).

5. Ophtalmoscope à balayage selon l'une des revendications précidéntes, caractérisé en ce qu'il comporte deux organes compensateurs de réfraction semblables ($B_a$, $B_r$) placés l'un sur le trajet d' illumination en amont de l'organe séparateur, l'autre sur le trajet d'observation en aval de l'organe séparateur, de façon à permettre une correction en fonction de l'amétropie éventuelle du patient.

6. Ophtalmoscope à balayage selon la revendication 5 caractérisé en ce que chaque organe compensateur ($B_a$, $B_r$) comporte un objectif fixe ($O_2$, $O_1$) et un chariot (BC) porteur d'un objectif mobile ($O_3$, $O_4$) et d'un diaphragme associé ($P_2$, $P'_3$ ou $P_3$), les chariots des deux organes compensateurs se déplaçant conjointement et solidairement.

7. Ophtalmoscope à balayage selon la revendication 6, caractérisé en ce que les organes compensateurs ($B_a$, $B_r$) sont prévus pour recevoir des verres cylindriques identiques au voisinage de leurs diaphragmes respectifs.

8. Ophtalmoscope à balayage selon l'une des revendications 6 et 7, caractérisé en ce que, dans chaque compensateur de réfraction, les objectifs fixe ($O_1$, $O_2$) et mobile ($O_3$, $O_4$) sont placés entre le séparateur de faisceau ($L_1$) et le diaphragme ($P_2$, $P_3$, $P'_3$) qui leur est associé.

9. Ophtalmoscope à balayage selon l'une des revendications 6 à 8, caractérisé en ce qu'à l'entrée du compensateur de réfraction associé au trajet d'illumination est prévue une letille ($O_6$ ou $O'_6$) renvoyant le point-source ($A_o$) produit par

le bloc source lumineuse (A) à l'infini.

10. Ophtalmoscope à balayage selon la revendication 9 caractérisé en ce que ladite lentille est interchangeable pour permettre le travail en microscopie rétinienne à balayage ($O_6$) ou en observation à champ moyen ($O'_6$), les diaphragmes des compensateurs de réfraction étant ajustés en conséquence ($P_3$, $P'_3$).

11. Ophtalmoscope à balayage selon l'une des revendications 1 à 10, caractérisé en ce que le bloc de source lumineuse (A) comprend un laser ($S_1$) ou un arc ($S_2$) et des moyens optiques à focalisation ($O_7$, $O_8$) pour produire à partir de celui-ci ledit point-source ($A_o$).

12. Ophtalmoscope à balayage selon la revendication 11, caractérisé en ce que les moyens optiques comprennent une fibre optique ($F_1$), au moins pour le travail en champ moyen, ce qui permet une séparation physique entre la source proprement dite et la partie de l'ophtalmoscope qui est rapprochée du patient.

13. Ophtalmoscope à balayage selon l'une des revendications 1 à 12, caractérisé en ce qu'un objectif de détection ($O_5$) est placé immédiatement en amont du petit diaphragme de détection (T), lequel est placé au foyer de cet objectif, et suivi du photodétecteur (PM).

14. Ophtalmoscope à balayage selon l'une des revendications 1 à 13, caractérisé en ce que le plus rapide ($M_4$) des deux déviateurs des moyens de balayage est oscillant, produisant ainsi un balayage sinusoïdal, et en ce que l'ophtalmoscope comporte des moyens électroniques (101-118) propres à numériser la sortie du photodétecteur (PM, 100), ainsi qu'à remplir en sens inverse deux mémoires numériques (102, 103) à l'aide des signaux détectes respectivement pendant l'aller et le retour d'une période de la sinusoïde.

15. Ophtalmoscope à balayage selon la revendication 14, caractérisé en ce que l'échantillonnage de la sortie du photodétecteur (PM) est effectué à une cadence variable en fonction de la vitesse d'excursion du balayage sinusoïdal.

## Patentansprüche

1. Ablenkophthalmoscop, bestehend aus Mitteln zum Beleuchten des Auges sowie Mitteln zur Beobachtung des solchermaßen beleuchteten Auges, wobei die Mittel zur Augenbeleuchtung einen Lichtquellenblock (A), Ablenkmittel (C) zum Ablenken des von dem Lichtquellenblock erzeugten Lichtstrahls entsprechend einem Zeilenraster und optische Mittel (D) aufweisen, die den auf das Auge des Patienten abgelenkten Lichtstrahl über die Pupille weiterleiten und zugleich im umgekehrten Sinn zur Beobachtung dienen, und wobei das genannte Ophthalmoscop weiterhin Mittel ($L_1$) zum Trennen der Lichtstrahlen bei der Beobachtung und bei dem Beleuchten aufweist und die Mittel zur Beobachtung einen fotoelektrischen Wandler, z. B. einen Fotomultiplier (PM) zum Erfassen des derart getrennten Beobachtungsstrahls aufweisen,

dadurch gekennzeichnet,

daß die Ablenkmittel (C) zugleich im umgekehrten Sinn zur Beobachtung dienen, daß die genannten Mittel ($L_1$ zum Trennen der Lichtstrahlen ($L_1$) aus einem Organ, wie einem Strahlteiler, bestehen, der in der Bahn des der Beobachtung dienenden Lichtstrahls strahlabwärts der Ablenkmittel (C) angeordnet ist, daß die Mittel zur Beobachtung desweiteren eine kleine Detektionsblende (T) aufweisen, die mit einer von dem Lichtquellenblock erzeugten Punktquelle ($A_o$) zusammenwirkt und deren Öffnung etwas über der Bildgröße der Punktquelle liegt, und daß der fotoelektrische Wandler strahlabwärts des Objektivs angeordnet ist.

2. Ablenkophthalmoscop nach Anspruch 1, dadurch gekennzeichnet, daß die zur Übertragung zum Auge dienenden optischen Mittel (D) Lichtreflexions-(Katoptrik)-mittel ($M_1$) sind, was die Unterdrückung von Störbildern gestattet.

3. Ablenkophthalmoscop nach Anspruch 2, dadurch gekennzeichnet, daß die zur Übertragung zum Auge dienenden optischen Mittel einen sphärischen Spiegel mit einem von Null abweichenden Einfallswinkel aufweisen, daß die Ablenkmittel (C) zwei angetriebene Strahlablenker ($M_3$, $M_4$) aufweisen, welche um entsprechende Rotationsachsen (x, y) arbeiten, die kolinear zu den Fokaldistanzen des Astigmatismus sind, die der sphärische Spiegel ($M_1$) aufgrund des von Null abweichenden Einfallswinkels auf dem punktartigen Bild der Patientenpupille erzeugt.

4. Ablenkophthalmoscop nach Anspruch 3, dadurch gekennzeichnet, daß die beiden angetriebenen Ablenker drehbare Spiegel ($M_3$, $M_4$) sind.

5. Ablenkophthalmoscop nach einem der vorhergehenden Ansprüche, dadurch gekennzeichnet, daß es zwei ähnliche Brechungskompensatoren ($B_a$, $B_r$) aufweist, von denen einer im Beleuchtungsstrahl strahlaufwärts des Strahlteilers, der andere im Beobachtungsstrahl strahlabwärts des Strahlteilers derart angeordnet ist, daß eine Korrektur in Abhängigkeit von einer eventuellen Weit- oder Kurzsichtigkeit des Patienten möglich ist.

6. Ablenkophthalmoscop nach Anspruch 5, dadurch gekennzeichnet, daß jeder Kompensator ($B_a$, $B_r$) ein ortsfestes Objektiv ($O_2$, $O_1$) und einen Schlittenträger (BC) für ein bewegliches Objektiv ($O_3$, $O_4$) und eine zugeordnete Blende ($P_2$, $P_3$, $P'_3$) aufweist, wobei die Schlitten der beiden Kompensatoren gemeinsam und voneinander abhängig bewegbar sind.

7. Ablenkophthalmoscop nach Anspruch 6, dadurch gekennzeichnet, daß die Kompensatoren ($B_a$, $B_r$) zur Aufnahme gleicher zylindrischer Gläser nahe ihrer entsprechenden Blenden eingerichtet sind.

8. Ablenkophthalmoscop nach einem der Ansprüche 6 und 7, dadurch gekennzeichnet, daß in jedem Brechungskompensator die ortsfesten Objektive ($O_1$, $O_2$) und die beweglichen Objektive ($O_3$, $O_4$) zwischen dem Strahlenteiler ($L_1$) und der diesem zugeordneten Blende ($P_2$, $P'_3$, $P_3$) angeordnet sind.

9. Ablenkophthalmoscop nach einem der Ansprüche 6 bis 8, dadurch gekennzeichnet, daß am Eingang des dem Strahleingang der Beleuchtung zugeordneten Kompensators eine Linse ($O'_6$, oder $O_6$) angeordnet ist, welche die von dem Lichtquellenblock (A) erzeugte Punktquelle ($A_0$) ins Unendliche zurückwirft.

10. Ablenkophthalmoscop nach Anspruch 9, dadurch gekennzeichnet, daß die genannte Linse austauschbar ist, um das Arbeiten in der Netzhaut-Ablenkmikroskopie ($O_6$) oder in der Mittelfeld-Beobachtung ($O'_6$) zu ermöglichen, wobei die Blenden der Brechungskompensatoren in Folge einjustiert sind ($P_3$, $P'_3$).

11. Ablenkophthalmoscop nach einem der Ansprüche 1 bis 10, dadurch gekennzeichnet, daß der Lichtquellenblock (A) einen Laser ($S_1$) oder einen Lichtbogen ($S_2$) und optische Fokusierungsmittel ($O'_7$, $O_8$) aufweist, um von diesem aus die genannte Punktquelle ($A_0$) zu erzeugen.

12. Ablenkophthalmoscop nach Anspruch 11, dadurch gekennzeichnet, daß die optischen Mittel zumindest für das Arbeiten im Mittelfeld eine optische Faser ($F_1$) aufweist, was ein physische Trennung zwischen der zuvor erwähnten Quelle und dem Teil des Ophthalmoscops, der sich nahe beim Patienten befindet, gestattet.

13. Ablenkophthalmoscop nach einem der Ansprüche 1 bis 12, dadurch gekennzeichnet, daß ein Detektionsobjektiv ($O_5$) unmittelbar strahlaufwärts der kleinen Detektionsblende (T) angeordnet ist, die sich am Brennpunkt dieses Objektivs befindet und von einem Fotodetektor (PM) gefolgt ist.

14. Ablenkophthalmoscop nach einem der Ansprüche 1 bis 13, dadurch gekennzeichnet, daß der schnellere ($M_4$) der beiden Ablenker der Ablenkmittel oszillierend ausgebildet ist und so ein sinusförmiges Ablenksignal erzeugt, und daß das Ophthalmoscop elektronische Mittel (101-118) sowohl zum Digitalisieren des Ausgangssignals des Fotodetektors (PM 100) als auch in umgekehrter Richtung zum Auffüllen von zwei nummerischen Speichern (102, 103) mit Hilfe der während des Vorlaufs und des Rücklaufs einer Sinusperiode entsprechend detektierten Signale.

15. Ablenkophthalmoscop nach Anspruch 14, dadurch gekennzeichnet, daß die Abtastung am Ausgang des Fotodetektors (PM) in variablem Takt in Abhängigkeit von der Auslenkgeschwindigkeit der sinusformigen Ablenkung erfolgt.

**Claims**

1. Scanning ophthalmoscope, comprising means for the illumination of the eye and also means for the observation of the eye thus illuminated, the said means for the illumination of the eye comprising a block of light source (A), scanning means (C) suited to deflect the beam produced by the block of light source according to a raster of lines, and optical means (D) for the transmission of the beam of light thus deflected to the eye of the patient, through his pupil, the said optical means of transmission (D) being likewise used, in the reverse direction, for observation, the said ophthalmoscope comprising in addition means ($L_1$) to separate the beams of observation and of illumination, and the said observation means comprising a photoelectric transducer means such as a photomultiplier (PM) to detect the observation beam thus separated, characterized in that the scanning means (C) are likewise used, in the reverse direction, for observation, in that the said means for separation of the beams ($L_1$) are constituted by a member such as a separating sheet, situated on the path of the observation beam below the scanning means (C), in that the observation means comprise in addition a small detection diaphragm (T) conjugate with a source point ($A_0$) produced by the block of light source, and with an opening slightly greater than the size of the image of this source point, and in that the said photoelectric transducer means is placed below the said diaphragm.

2. Scanning ophthalmoscope according to claim 1, characterized in that the optical means for transmission to the eye (D) are of the catoptric type ($M_1$), which permits spurious images to be eliminated.

3. Scanning ophthalmoscope according to claim 2, characterized in that the optical means for transmission to the eye comprise a spherical mirror ($M_1$), which is used under a non-zero incidence, and in that the scanning means (C) comprise two controlled beam deflectors ($M_3$, $M_4$) operating about respective axes of rotation (x, y) colinear to the focal lines of astigmatism which the spherical mirror ($M_1$) would produce on the punctual pupil image of a patient, taking into account the said non-zero incidence.

4. Scanning ophthalmoscope according to claim 3, characterized in that the two controlled deflectors are pivoting mirrors ($M_3$, $M_4$).

5. Scanning ophthalmoscope according to one of the preceding claims, characterized in that it comprises two similar refraction compensating members ($B_a$, $B_r$) one placed on the illumination path above the separating member, the other on the observation path below the separating member, so as to permit a correction as a function of the possible ametropia of the patient.

6. Scanning ophthalmoscope according to claim 5, characterized in that each compensating member ($B_a$, $B_r$) comprises a fixed objective ($O_2$, $O_1$) and a carriage (BC) bearing a movable

objective ($O_3$, $O_4$) and an associated diaphragm ($P_2$, $P'_3$ or $P_3$), the carriages of the two compensating members moving jointly and integrally.

7. Scanning ophthalmoscope according to claim 6, characterized in that the compensating members ($B_a$, $B_r$) are provided to receive identical cylindrical glasses in the vicinity of their respective diaphragms.

8. Scanning ophthalmoscope according to one of claims 6 and 7, characterized in that, in each refraction compensator, the fixed ($O_1$, $O_2$) and movable ($O_3$, $B_4$) objectives are placed between the beam separator ($L_1$) and the diaphragm ($P_2$, $P_3$, $P'_3$) which is associated therewith.

9. Scanning ophthalmoscope according to one of claims 6 to 8, characterized in that at the input of the refraction compensator associated with the illumination path there is provided a lens ($O_6$ or $O'_6$) returning the source point ($A_o$) produced by the block of light source (A) to infinity.

10. Scanning ophthalmoscope according to claim 9, characterized in that the said lens is interchangeable to permit scanning retinal microscopy work ($O_6$) or mid-field observation work ($O'_6$), the diaphragms of the refraction compensators being adjusted as a result ($P_3$, $P'_3$).

11. Scanning ophthalmoscope according to one of claims 1 to 10, characterized in that the block of light source (A) comprises a laser ($S_1$) or an arc ($S_2$) and optical means for focussing ($O_7$, $O_8$) to produce the said source point ($A_o$) therefrom.

12. Scanning ophthalmoscope according to claim 11, characterized in that the optical means comprise an optical fibre ($F_1$), at least for mid-field work, which permits a physical separation between the actual source and the part of the ophthalmoscope which is brought close to the patient.

13. Scanning ophthalmoscope according to one of claims 1 to 12, characterized in that a detection objective ($O_5$) is placed immediately above the small detection diaphragm (T), which is placed in the focus of this objective, and followed by the photodetector (PM).

14. Scanning ophthalmoscope according to one of claims 1 to 13, characterized in that the fastest ($M_4$) of the two deflectors of the scanning means is oscillating, thus producing a sinusoidal scanning, and in that the ophthalmoscope comprises electronic means (101-118) suited to numerise the outlet of the photodetector (PM 100), and also to fill in reverse direction two numerical memories (102, 103) by means of the signals detected respectively during the outgoing and returning of a period of the sinusoid.

15. Scanning ophthalmoscope according to claim 14, characterized in that the sampling of the outlet of the photodetector (PM) is effected at a cadence which is variable as a function of the speed of excursion of the sinusoidal scanning.

FIG.1

FIG. 2

FIG. 3

ECRITURE
MEMOIRE

LECTURE
MEMOIRE

0 145 563

FIG. 4

## FIG. 5

VERS 106 (FIG.4)

ECR1    ECR 2

LEC1    LEC2

VERS 107 (FIG.4)

ARRET DU COMPTAGE
SENS DE COMPTAGE
ET REMISE A ZERO DU
COMPTEUR 107

(A.,B.C.,D)
DE

112 (FIG.4)

LOGIQUE
DE
COMMANDE

SYNC

VERS 114
(FIG.4 OU 6)

113

## FIG. 6

114

Lsync

VERS 104 (FIG.4)

SYNCHRO TRAME
VERS 115 (FIG.4)
SYNCHRO LIGNE
VERS 108 (FIG.4)

130

131

VCO

1MHz

132

UNITE VIDEO
(S178A)

64µs

9